Europäisches Patentamt

(19) European Patent Office

Office européen des brevets

(11) Publication number: **0 244 998**
**B1**

## EUROPEAN PATENT SPECIFICATION

(45) Date of publication of the patent specification:
07.02.90

(51) Int. Cl.⁴: **B01D 15/08**, C12N 1/02

(21) Application number: 87303693.3

(22) Date of filing: 27.04.87

(54) **Method for clarifying and stabilizing cell-culture media.**

(30) Priority: 28.04.86 US 854984
16.04.87 US 37307

(43) Date of publication of application:
11.11.87 Bulletin 87/46

(45) Publication of the grant of the patent:
07.02.90 Bulletin 90/6

(84) Designated Contracting States:
AT BE CH DE ES FR GB IT LI NL SE

(56) References cited:
EP-A- 0 009 394
AU-A- 509 624
FR-A- 2 359 634
FR-A- 2 541 289
GB-A- 896 439
US-A- 2 684 322
US-A- 3 547 900

(73) Proprietor: ROHM AND HAAS COMPANY, Independence Mall West, Philadelphia Pennsylvania 19105(US)

(72) Inventor: Byers, Michael Joe, P.O. Box 274, Gwynedd Pennsylvania 19436(US)
Inventor: Isacoff, Eric Gilbert, 9 Juniper Drive, Richboro Pensylvania 19436(US)
Inventor: Kim, Chan Wha, 402 Rindge Avenue, No. 4A, Cambridge Massachusetts 02140(US)
Inventor: Naples, John Otto, 1570 Dreshertown Road, Dresher Pennsylvania 19025(US)
Inventor: Robinson, Elizabeth Marie, 929 Mass Avenue, No. 6E, Cambridge Massachusetts 02139(US)
Inventor: Rha, Chokyun, 285 Commonwealth Avenue, Boston Massachusetts 03115(US)

(74) Representative: Tanner, James Percival et al, ROHM AND HAAS (UK) LTD. European Operations Patent Department Lennig House 2 Mason's Avenue, Croydon CR9 3NB(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention is concerned with a method for simultaneously clarifying and stabilizing liquid, cell-culture media such as fermentation broths.

In the long history of microbiology, many substances have been produced by fermentation. Antibiotics, proteins, organic acids, alcohols, and other carbohydrates to say nothing of wines and beers, have been produced in this way. In these fermentations, a carefully selected microorganism is grown in a liquid medium containing the proper nutrients. The product is then harvested by separating the liquid portion of the medium, which contains the product, from the solid matter which is mostly cells, cell debris and spent nutrients. This separation has usually been carried out by centrifugation or filtration.

The needs of commerce usually dictate that the supernatant liquid or filtrate be clear. Frequently, however, the cell-culture medium contains (or the organisms produce) a contaminating proteinaceous or other material which generates haze and/or color in the product-containing liquid, either on filtering, on later standing or during subsequent processing. For commercial acceptability of the product, such hazy material and/or color bodies, or generators thereof, must ordinarily be removed and the medium stabilized against further color and/or haze generation. This has been done by one or more "polishing" steps, such as coagulation with calcium chloride. However, such "polishing" steps are time consuming, expensive and often result in substantial product loss due to tight binding of product in a gelatinous precipitate.

In this specification, the expression "cell-culture medium" means the fermentation broth, i.e., the liquid medium in which microorganisms are grown, or filtrates or liquid fractions of broths, including or excluding cells, as well as cell debris and other material resulting from lysis, if practiced.

We have surprisingly found that a hazy and/or coloured liquid, cell-culture medium containing one or more of (i) biological cells and/or cell debris, (ii) spent nutrients and (iii) contaminating water-soluble haze- and/or color-generating proteinaceous materials can be simultaneously clarified and stabilized to give a clear, color- and/or haze-free liquid product. These benefits are achieved by treating the medium with a particulate polymer having anion, and optionally cation, exchange funcionality with a selected particle diameter either alone or in conjunction with a water-soluble polymer. This enables a reduction of further purifying operations, increased yield of product, and significant savings in time and expense to be achieved when compared to known procedures.

According to the present invention there is provided a method for simultaneously clarifying and stabilizing a hazy and/or coloured liquid, cell-culture medium comprising desired product and one or more of (i) biological cells and/or cell debris, (ii) spent nutrients, and (iii) contaminating water-soluble haze- and/or color-generating proteinaceous materials, characterized in that the method comprises:-

(1) adding to cell-culture medium an effective amount of:
(a) particulate, polymer having acion, and optionally cation, exchange functionally and a particle diameter of 0.01 to 50 micrometers, a optionally
(b) water-soluble polymer; and
(2) separating solids from the material obtained from step (i) to give a clear, colour and/or haze-free, liquid containing the desired product.

The particulate polymer used in the method of the invention can be any particulate polymer having anion, and optionally cation, exchange functionally. These materials bind or otherwise hold the color- and/or haze-generators by chemical or electrostatic means. Useful particulate polymers include ion exchange resin precursors (preferably crosslinked but also including water-insoluble, uncrosslinked materials).

By combination of a water-soluble, functional polymeric material, such as an acrylic polymer, with the water-insoluble, particulate polymer having anion, and optionally cation, exchange functionality, the efficiency of removal of the haze- and/or color-generating substances may be greatly improved. Thus, for example, the same overall effect may be obtained when using 2000 ppm of insoluble (particulate), charged polymeric adsorbent alone as is obtained when using about 1200 ppm of the same adsorbent combined with about 30 ppm of water-soluble acrylic polymer (e.g. 65 wt.% methacrylic acid/35 wt.% ethyl acrylate). The functional group of the acrylic polymer generally has one or more of hydroxyl, carboxyl, amino or amide functional groups. Typical of monomers having such functionality are amides such as acrylamide, methacrylamide, N-methylolacrylamide, N-monoalkyl and N-dialkyl acrylamides; tertiary amino compounds such as dialkylaminoalkyl acrylates and methacrylates such as dimethylaminoethyl methacrylate; and hydroxyalkyl acrylates and methacrylates such as hydroxyethyl acrylate. Such monomers are usually copolymerized with other acrylic monomers, including the various alkyl acrylates, alkyl methacrylates, acrylic acid and methacrylic acid.

The particle size of the particulate polymer is 0.01 to 50 micrometers. If the particles are smaller than 0.01 µm, they tend to clog filters and slow the filtration process; if they are larger than 50 µm, there will be incomplete capture of the haze/color-generators. The particle size is preferably 0.01 to 50 micrometers, 0.05 to 2 micrometers. In the case of irregularly shaped particles (e.g., ground resins), the above-mentioned particle diameters are, for the purposes of this invention, assumed to refer to the longest dimensions of the particles.

As indicated above, the particulate, polymer useful in the method of this invention preferably are crosslinked and more preferably they are uniformly functionalized, as conventional for materials available heretofore in the ion exchange field. However, water- insoluble, uncrosslinked materials may also be suitable. For example, ion exchange polymer particles functionalized with an ionogenic group near the particle surface, e.g. a monolayer of ion exchange groups about the periphery of the bead, are useful. Lightly crosslinked or surface-crosslinked beads having low water solubility are also effective.

The particular type of resin to be used is dictated by the nature of the material to be removed, for example haze- and/or color-generating material and can be readily selected by one skilled in this art using well-known principles of ion exchange chemistry, based on routine determinations of functionality and charge density of material to be removed by the treatment. Of course, one must use care to select a resin which, while removing the materials, e.g. haze/color-generators, will not substantially bind the desired product.

Depending on the material to be removed, the ion exchange resin may be a single resin containing anion exchange functionality, a single resin containing both anionic and cationic functionality (amphoteric resin), or may be a mixed, hybrid, chelating or composite resin having anionic or both anionic and cationic exchange character. Furthermore, both gel and macroreticular resins are useful, although the macroreticular resins are preferred because of their high porosity and the consequent greater opportunity for adsorbing impurities from the culture media as well as exchanging with ionic species which may be contributing to haze and/or color in the media. Ion exchange resins may be used as beads, ground material, powders, or other particulate form. If desired, treatment with a positively charged adsorbent may be followed by treatment with a negatively charged adsorbent.

Ion exchange resins useful in the invention include the macroreticular vinyl aromatic or acrylic resin adsorbents and exchangers described in US-A-3,037,052, 3,637,535, 3,843,566, 3,791,866, 3,275,548 and 3,357,158, the hybrid resins described in US-A-3,991,017, the composite resins described in US-A-3,645,922 and the amphoteric resins described in US-A-4,202,737. The disclosures of these patents are incorporated herein by reference.

As is well known, particulate ion exchange resins may be prepared by first forming a crosslinked copolymer matrix (usually by suspension polymerization but alternatively by emulsion polymerization as in US-A-4,380,590) and then functionalizing the copolymer particles to provide the requisite ion exchange capacity. In such manner, individual weakly or strongly basic ion exchange resins may be prepared, and then used, in the present invention, singly or in admixture, with or without water-soluble, polymer.

The particulate, polymers preferred for use in the invention are those described in US-A-4,200,695 to Chong, Isacoff and Neely, 4,359,537 to Chong, and 4,537,683 to Isacoff and Neely. The disclosures of these patents are incorporated herein by reference. The polymers of the foregoing patents are, generally speaking, ion exchange resins composed of crosslinked polymers in the shape of spherical beads, produced by emulsion polymerization. These resins have the small particle size preferred for use in this invention and bear about 0.1 to 1.5 functional groups per monomer unit, defining ion exchange capacity (charge density), which groups can be strongly basic (e.g. quaternary ammonium groups), or weakly basic (e.g. tertiary amine groups). Ion exchange resins having other functional groups and ion exchange capacities can also be used.

The liquid medium in which microorganisms are grown may be conventional and may contain nutrients which supply sources of carbon and nitrogen, as well as the various salts and accessory factors needed for vigorous growth and fermentation. The pH is conveniently adjusted to a range best suited for maximum growth and production. The medium may also contain, either before fermentation begins or afterward, material which generates haze and/or color.

This medium can be inoculated with a culture of the desired organism, for example, a fungus, yeast or bacterium, and then incubated at a suitable temperature, under aerobic or anaerobic conditions as necessary, until the fermentation is complete. If the desired product is intracellular, the microbial cells must be lysed to release it. The procedure may also be applied with obvious modifications, to other processes, as for example continuous fermentation, which will be apparent to those skilled in the art.

The method of the invention may then be performed, but can be performed on fractions or portions of the medium, including supernatants obtained by preliminary separation processes.

The polymer is added to the liquid medium, which is then stirred or otherwise agitated for a suitable period, e.g. for about 5 to 30 minutes. The amount of polymer used is a practical matter: if too much is used, the medium becomes thick and hard to manage; if too little is used, there is an insufficient haze- and/or color-eliminating and stabilizing effect. Ordinarily from about 0.01 to about 10 weight %, per volume of liquid cell-culture medium, e.g. broth, will give satisfactory results, but other amounts and proportions may be used.

The cell-culture, medium solids and liquid are then separated, ordinarily by filtration and/or centrifugation. The resulting filtrate/supernatant liquid is a clear, essentially colorless and haze-free liquid containing the product.

Although the foregoing describes the method of the invention used with microbial cultures, it is to be understood that it can also be used with the same or equivalent effects on liquors obtained by the culture of animal or other plant cells.

The present invention will now be further illustrated by way of the following examples.

In the Examples, all parts and percentages are by weight unless otherwise specified, and the polymer samples used have the compositions given in Table 1, Sample D (a cation exchanger) being included for comparison with the polymers of the invention:

## Table 1

| Polymer sample | |
| --- | --- |
| A. | Quaternary amine functionalized, styrene/divinylbenzene cellular copolymer, containing 1.8% divinylbenzene crosslinker. Anion Exchange Capacity (AEC) = 3.8 meq/g dry; particle diameter = 0.22±0.02 micrometer. |
| B. | Styrene/divinylbenzene/aminoalkyl methacrylate cellular copolymer, containing 5% divinylbenzene crosslinker and in which the tertiary amine functionality has been quaternized. AEC = 2.8meq/g dry; particle diameter = 0.11±0.02 micrometer. |
| C. | Styrene/divinylbenzene/aminoalkyl methacrylate cellular copolymer, containing 5% divinylbenzene crosslinker and having tertiary amine functionality. AEC = 3.3 meq/g dry; particle diameter = 0.09±0.02 micrometer. |
| D. | Sulfonic acid functionalized, styrene/divinylbenzene cellular copolymer, containing 7.3% divinylbenzene crosslinker. Cation exchange capacity (CEC) = 5.1 meq/g dry; particle diameter = 0.26±0.02 micrometer. |
| E. | High molecular weight, water-soluble polyacrylamide anionic polymer marketed as Polymer LT 27 by Allied Colloids. |
| F. | Anionic water-soluble polymer prepared from 65 wt.% methacrylic acid and 35 wt.% ethyl acrylate. |
| G. | Quaternary amine functionalized, styrene/divinylbenzene macroreticular resin ground to an average particle size of 1 micrometer. |

## EXAMPLE 1

An overnight culture of Saccharomyces cerevisiae (Baker's Yeast) was thoroughly mixed with 0.1%, based on the weight of the culture, of Sample A. To this mixture was added 0.1%, based on the weight of the culture, of Sample D and the vessel inverted. A floc formed immediately and the yeast cells settled rapidly to the bottom of the vessel. The cells retained viability as determined by the continued evolution of gas from the cell pellet, formed upon settling of the floc, and the supernatant was free of haze and essentially colorless.

## EXAMPLE 2

Separately, 2% and/or 3% aqueous suspensions of Saccharomyces cerevisiae (Baker's yeast) were mixed for one minute with 0.1% aqueous suspensions of resin Samples A to D. The suspensions were then centrifuged for 10 minutes at 700 × g., with the following results:

## Table 2

| | |
| --- | --- |
| (a) | The mixture of the 0.1% aqueous suspension of Sample A and 2% aqueous suspension of yeast yielded a clear supernatant with an increase in precipitate volume over the yeast alone. |
| (b) | The mixture of the 0.1% aqueous suspension of Sample B and 3% aqueous suspension of yeast yielded a clear supernatant with an increase in precipitate volume over the yeast alone. |
| (c) | The mixture of the 0.1% aqueous suspension of Sample C and 2% aqueous suspension of yeast yielded a clear supernatant with an increase in precipitate volume over the yeast alone. |
| (d) | The mixture of the 0.1% aqueous suspension of Sample D and the 2% or 3% aqueous yeast suspensions failed to yield a clear supernatant. |

This example thus demonstrates that positively charged, fine particle size ion exchange resins (Samples A,B,C) electrostatically bind to negatively charged yeast cells and provide clear supernatants whereas a negatively charged fine particle size ion exchange resin (Sample D) does not.

## EXAMPLE 3

To determine sedimentation rates of yeast cells with various adsorbents, a 0.5% aqueous suspension of Saccharomyces cerevisiae was prepared and 0.1%, based on the weight of the yeast suspension, of ion exchange resin was added. The mixture was shaken for 20 minutes and the sedimentation volume measured as a function of time as shown below:

Table 3

| Resin sample | Maximum sedimented volume (ml.) | Time (min.) |
|---|---|---|
| A | 10.0 | 2.5 |
| B | 3.5 | 3.5 |
| C | 2.5 | 11.0 |

The results show that Sample A has the highest affinity for yeast cells. The supernatants resulting from the mixture were haze-free and essentially colorless.

<u>EXAMPLE 4</u>

<u>Bacillus Licheniformis</u> was prepared in a broth of 5% starch, 1% glucose, 1% soybean meal, 1% casami-no acids, 0.5% ammonium phosphate and 0.05% magnesium sulfate, the percentages being based on the weight of broth. After 72 hours incubation, the dry cell weight per liter was 18 g. Then 10 ml of cell suspension was made to 0.1% with respect to the resin and shaken in test tubes and velocities, pellet volume and g biomass/g adsorbent resin measured. As seen in the table below, the strong base resins have the highest capacity and Sample A has the fastest sedimentation rate. The supernatants were haze-free and essentially colorless.

Table 4

| Resin sample | Sedimentation velocity (Cm/hr) | g Biomass/ g resin | Pellet volume (cc) |
|---|---|---|---|
| A | 0.92 | 16.6 | 0.48 |
| B | 0.40 | 17.8 | 0.76 |
| C | 0.71 | 6.3 | 0.40 |
| D | 0.61 | 11.5 | 0.40 |
| Blank | — | 0 | 0.36 |

<u>EXAMPLE 5</u>

The effect of combining particulate and water soluble polymers on clarification of a culture which is a supernatant (20% solids) containing alkaline protease extracellular enzyme was studied, as compared with conventional $CaCl_2$ flocculation.

The broth treated was a 50% culture/50% water. To 800 ml of the 50/50 broth in 250 ml beakers was added the polymer(s). The mixtures were homogenized and then centrifuged at 1000 × g for 5 minutes. Turbidity of the supernatant was determined at 450 nm, with results as follows, wherein turbidity is reported in Formazin Turbidity Units (FTU) at 450 nm.

Table 5

| Adsorbents | | Results after centrifugation | |
|---|---|---|---|
| | | Turbidity (FTU) | floc volume (ml) |
| (a) | 30,000 ppm $CaCl_2$ + 5,000 ppm $NaH_2PO_4$ + 250 ppm sample E | 2,000 | 400 |
| (b) | 2000 ppm sample A, 50 ppm sample F | 1,500 | 300 |
| (c) | 2000 ppm sample A, 500 ppm sample D | 3,500 | 80 |
| (d) | 750 ppm sample A, 20 ppm sample F | 3,000 | 250 |
| (e) | 4000 ppm sample A, 200 ppm sample F | 1,000 | Not measured |
| (f) | Blank | 60,000 | — |

All of the supernatants appeared fairly clear, but color interfered somewhat with the turbidity measurement at 450 nm. The results show excellent settling rates and clarity (lowered turbidity) for the 2000/50 ppm dosages of Samples A and F (run [b]). In run (c) there were three layers after centrifugation: a small supernatant, a large loose floc area, and a settled floc (80 ml).

EXAMPLE 6

Saccharomyces cerevisiae cells, 1 kg, were lysed by the slow addition of 2 liters of 0.12 M NH₄OH containing 2 mM of ethylene diamine tetraacetic acid. The solution was stirred for 4.5 hours at room temperature, after which the slurry was adjusted to pH 4.5 with 2M acetic acid. Distilled water was added to give a final volume of 7.5 liters. The solution was then stored at 4°C. To each of three 25 ml portions of the lysate in separate 50 ml centrifuge tubes were then added a resin (Sample A being added to one portion, Sample B being added to a second portion, and Sample C being added to the third portion). Each mixture was shaken, allowed to stand for five minutes at 4°C, and then centrifuged at 4000 × g for five minutes at 4°C. After centrifugation, the supernatant was removed and its turbidity measured by absorbance at 530 nm. The resins gave a reduction in turbidity to 10% of the control. These results indicate that the supernatants were extremely clear, with optimum ratios of resin to dry cell weight as follows:

Table 6

| Optimal concentration ranges for clarification of yeast cell debris | | | | |
|---|---|---|---|---|
| Resin sample A | 0.04 | to | 0.1 g | resin/g dry cell weight |
| Resin sample B | 0.01 | to | 0.02 g | resin/g dry cell weight |
| Resin sample C | 0.01 | to | 0.04 g | resin/g dry cell weight |

EXAMPLE 7

Ion exchange resin samples were added to 35 ml of Aerococcus lysate in 50 ml centrifuge tubes at room temperature and shaken. They were then centrifuged at 300 × g for 5 minutes at which time the supernatant was measured for turbidity (by absorbance at 530 nm) and enzyme activity. Cell-free lysates were used as controls. The results are set forth below from which it can be seen that Sample A provided the greatest clarification (decrease in turbidity) and least effect on enzyme activity.

Table 7

| Sample concentration (g sample/g cells) | Turbidity of supernatant (a.u.)[2] | Decrease in turbidity (%) | Enzyme activity % |
|---|---|---|---|
| 0.00 | 21.7 | 0.0 | 100 |
| Sample A | | | |
| 0.26 | 8.13 | 62.5 | 100 |
| 0.4 | 5.31 | 70.9[1] | 96 |
| 0.5 | 6.65 | 69.3 | n.m.[3] |
| 0.64 | 7.21 | 66.8 | n.m.[3] |
| Sample B | | | |
| 0.26 | 2.74 | 87.4 | n.m.[3] |
| 0.4 | 2.05 | 90.6[1] | n.m.[3] |
| 0.5 | 2.05 | 90.6 | n.m.[3] |
| Sample C | | | |
| 0.26 | 3.22 | 85.2 | 95 |
| 0.4 | 1.58 | 92.7[1] | 69 |
| 0.5 | 1.75 | 91.9 | n.m.[3] |

[1] Optimal concentration of sample as determined by turbidity.
[2] Absorbance units.
[3] Not measured.

EXAMPLE 8

The experiment of Example 7 was repeated, using Flavobacterium lysate in place of Aerococcus. The results (below) are similar to those of Example 7.

Table 8

| Sample concentration (g sample/g cells) | Turbidity of supernatant (a.u.)[2] | Decrease in turbidity (%) | Enzyme activity % |
|---|---|---|---|
| 0.00 | 62.0 | 0.0 | 100 |
| Sample A | | | |
| 0.12 | 61.43 | 1.0 | 93 |
| 0.23 | 10.7 | 82.7 | 90 |
| 0.35 | 9.1 | 85.3 | 90 |
| 0.46 | 9.01 | 85.5[1] | 90 |
| Sample B | | | |
| 0.12 | 31.3 | 49.5 | n.m.[3] |
| 0.23 | 11.54 | 81.4 | n.m.[3] |
| 0.35 | 9.71 | 84.3[1] | n.m.(3) |
| Sample C | | | |
| 0.12 | 12.55 | 79.8 | 100 |
| 0.23 | 10.91 | 82.4 | 97 |
| 0.35 | 10.00 | 83.8[1] | 92 |

[1] Optimal concentration of sample as determined by turbidity.
[2] Absorbance units.
[3] Not measured.

## EXAMPLE 9

The experiment of Example 7 was repeated, using Saccharomyces cerevisiae (Baker's Yeast) lysate in place of Aerococcus, with results similar to those of Examples 7 and 8.

Table 9

| Sample concentration (g sample/g cells) | Turbidity of supernatant (a.u.)[2] | Decrease in turbidity (%) | Enzyme activity % |
|---|---|---|---|
| 0.00 | 17.3 | 0.0 | 100 |
| Sample A | | | |
| 0.01 | 8.03 | 53.6 | 100 |
| 0.02 | 7.54 | 56.4[1] | n.m.[4] |
| 0.07 | 100.6 | −481.5[3] | n.m.[4] |
| Sample B | | | |
| 0.005 | 17.5 | −1.2 | n.m.[4] |
| 0.01 | 15.6 | 9.8[1] | n.m.[4] |
| 0.02 | 17.5 | −1.2 | n.m.[4] |
| 0.03 | 17.7 | −2.3 | n.m.[4] |
| Sample C | | | |
| 0.005 | 18.0 | −4.0 | 100 |
| 0.01 | 15.0 | 13.3[1] | n.m.[4] |
| 0.02 | 15.2 | 12.1 | n.m.[4] |
| 0.03 | 16.3 | 5.8 | n.m.[4] |

[1] Optimal concentration of sample as determined by turbidity.
[2] Absorbance units.
[3] Excess resin stabilized cell debris and remained suspended after centrifugation.
[4] Not measured.

EXAMPLE 10

One major problem encountered in the preparation of Malate Dehydrogenase (MDH) is clarification of the initial pig heart homogenate. This is partially due to the limit on the g forces obtainable within most production plants. Poorly clarified homogenate can lead to problems with subsequent process steps especially chromatography. This example illustrates clarification of pig heart homogenate using the method of the present invention. A 100-g sample of diced pig heart was blended with 100 ml ice water in a Waring Blender at full speed for 1 minute. This homogenate was centrifuged at 3,600 × g for 20 minutes at 4°C. The resulting supernatant was filtered through a cotton mesh filter to remove any large particulate matter, and was stored at 4°C until used. To four samples of pig heart homogenate were added 1000 and 5000 ppm Resin B and Resin C, respectively; no resin was added to a fifth, control sample:

Sample 1 – 1,000 ppm Resin B
Sample 2 – 5,000 ppm Resin B
Sample 3 – 1,000 ppm Resin C
Sample 4 – 5,000 ppm Resin C
Sample 5 – Control

Following addition of the resin, the samples were centrifuged at 3,250 × g for 4 minutes, and the supernatant was measured for turbidity and enzyme activity. The results of those measurements are shown in Table 10, below. Treated samples 1, 2 and 4 showed improved clarity compared to the control (sample 5), while all the treated samples showed a slight (2 to 4%) improvement in malate dehydrogenase activity compared to the control sample.

8

### Table 10

| Sample No. | Turbidity of supernatant at 530 nm | Enzyme activity (units/ml) |
|---|---|---|
| 1 | 0.064 | 470 |
| 2 | 0.066 | 480 |
| 3 | 0.410 | 480 |
| 4 | 0.067 | 480 |
| 5 | 0.410 | 460 |

The enzyme activity of the malate dehydrogenase was measured by following the oxidation of the reduced form of nicotinamide adenine dehydrogenase (NADH) spectrophotometrically at 340 nm during the reaction:

$$oxalacetate + NADH + H+ \rightarrow 1\text{-malate} + NAD+$$

and calculating the activity units:
Activity (units/ml) = ((Absorbance Change/minutes) × assay volume × dilution factor)/(6.22 × sample volume). Typical values for this determination are an assay volume of 3 ml, a sample volume of 0.1 ml, and a dilution factor of 1:100.

### EXAMPLE 11

Dry active yeast obtained from Genzyme Biologicals Ltd. was lysed by the addition of 0.12M $NH_4OH$. Resin G was added to 10 ml aliquots of yeast lysate at concentrations ranging from 1,000 ppm to 10,000 ppm. The solution was mixed on a rotary mixer for 10 seconds, allowed to equilibrate for 1 minute, and mixed for an additional 10 seconds. The mixture was then centrifuged for 2.5 minutes at 350 × g. The resulting supernatant was separated; its volume was measured and its turbidity was determined spectrophotometrically at 530 nm. The pellet of cell debris, formed upon centrifugation, was re-centrifuged for 15 minutes at 350 × g to completely pack the pellet. The supernatant was again collected and its volume determined. The controls used in this study were an untreated sample and a sample treated with 5,000 ppm Resin B. These controls were tested simultaneously with Resin G.

The results of this study are presented in Table 11 below. The optimum concentration for both Resins G and Resin B was determined to be approximately 5,000 ppm.

### Table 11

| | Resin type | Resin concentration | Turbidity* at 530 nm |
|---|---|---|---|
| 1. | Control | 0 | 1.353 |
| 2. | Resin B | 5,000 | 0.502 |
| 3. | Resin G | 1,000 | 3.908 |
| 4. | Resin G | 2,500 | 2.143 |
| 5. | Resin G | 5,000 | 0.856 |
| 6. | Resin G | 10,000 | 2.502 |

* Turbidity measured after first centrifugation.

### EXAMPLE 12

Maltase is an intracellular enzyme of yeast. The first step in its purification is cell lysis, followed by a clarification step to remove cell debris; this clarification step has typically been a centrifugation. This example illustrates the effect of a resin of the present invention upon the maltase clarification step. Resin B was added to a sample of yeast cell lysate at a level of 4000 ppm, based on the total lysate volume, and the mixture and a control sample without the resin were centrifuged at 3000 × g for 10 minutes. Maltase activity of the two samples was determined by spectrophotometrically measuring their hydrolysis of p-nitrophenylglucoside. The protein content of the samples was determined using the Lowry assay, and carbohydrate was determined using the phenol-sulfuric acid assay. The results of these tests are shown in Table 12.

**Table 12**

| Component assayed | Control | Treated sample | Recovery |
|---|---|---|---|
| Maltase activity (units/ml) | 171 | 164 | 96% |
| Protein (mg/ml) | 9.7 | 7.4 | 76% |
| Specific activity (units/mg protein) | 17.6 | 22.1 | 125% (purification) |
| Carbohydrate (mg/ml) | 5.0 | 2.1 | 42% |
| Turbidity (OD530 nm) | 0.094 | 0.075 | – |
| Colored components (OD410/OD365 nm) | 1.62 | 1.11 | – |

As may be seen from the table, adding Resin B to the sample benefitted the maltase purification in three ways. Contaminating non-maltase proteins were selectively removed with the cell debris, allowing a 25% increase in the maltase specific activity. The carbohydrate content of the clarified lysate was reduced by more than 50%, and the concentration of unidentified color-introducing components, which bind tightly to the column, and whose concentration was estimated by their absorbance at 410 nm, was reduced by 30%. As the contaminants removed by the addition of Resin B must normally be removed during later purification steps, their removal at this early stage facilitates subsequent purification of the maltase.

EXAMPLE 13

This example illustrates the removal of deoxyribonucleic acid (DNA) from solution using the polymeric particles of the present invention.

DNA from calf thymus (Sigma Chemical Co., Lot No. 105F-9530) was used to prepare a solution containing 2.5 mg/ml DNA in a buffer containing 35 mM sodium acetate and 1 mM neutralized disodium ethylenediamine tetracetic acid (EDTA) per liter and having a pH of 5.0.

This solution was diluted with buffer to prepare a set of five solutions at each of four DNA concentrations, as indicated in Table 13, below. To each set of solutions Resin B was added at the weight ratios of Resin B:DNA indicated in Table 13. Each solution was mixed on a vortex mixer and centrifuged, and the absorbance of the supernatant at 260 nm was determined for each.

As the table indicates, regardless of the initial concentration of DNA, a resin concentration of four times the DNA concentration removed essentially all the DNA from the solution. For resin concentrations below this optimum level, the higher absorbance results from remaining DNA color in the solution, and indicates that not all the DNA was removed. At higher resin concentrations the increase in absorbance above the minimum is caused by residual turbidity from the excess resin.

**Table 13**

| Resin: DNA ratio | DNA concentration (μg/ml) | | | |
|---|---|---|---|---|
| | 12 | 25 | 50 | 100 |
| | Solution absorbance at 260 nm | | | |
| 1:1 | 0.5 | 0.9 | 1.7 | 3.0 |
| 2:1 | 0.4 | 0.55 | 0.75 | 2.1 |
| 4:1 | 0.0 | 0.0 | 0.0 | 0.0 |
| 6:1 | 0.05 | 0.05 | 0.4 | 0.9 |
| 8:1 | 0.1 | 0.13 | 0.9 | 1.7 |

EXAMPLE 14

This example, which parallels Example 13, illustrates the removal of ribonucleic acid (RNA) from solution using the polymers of the present invention.

Three different buffer solutions were used in this example: Buffer A was the sodium acetate buffer of Example 13; Buffer B was a 50 mM per liter sodium potassium phosphate buffer having a pH of 7.8 and containing 1 mM per liter of neutralized EDTA; and Buffer C, which was actually unbuffered, deionized water containing 1 mM per liter of neutralized EDTA.

Solutions of 25 μg/ml RNA in each of the three buffers were prepared, and five samples of each RNA solution were treated with the amounts of Resin B indicated in Table 14, below. The treated samples were centrifuged and the supernatants were measured for absorbance at 260 nm. The results are shown in Table 14.

As these results show, Resin B effectively removes a significant portion of the RNA present in the solutions. Unlike the DNA in Example 13, however, some RNA remains in the solution even at the optimum treatment level.

### Table 14

| Resin concentra-tion (µg/ml) | Solution absorbance at 260 nm | | |
|---|---|---|---|
| | Buffer A | Buffer B | Buffer C |
| 0 | 0.40 | 0.40 | 0.40 |
| 5 | 0.40 | 0.40 | 0.40 |
| 10 | 0.30 | 0.35 | 0.50 |
| 20 | 0.10 | 0.25 | 0.10 |
| 40 | 0.25 | 0.40 | 0.25 |

### EXAMPLE 15

The two preceding examples showed that Resin B could completely remove DNA from solution, but only partially remove RNA. This example illustrates that Resin B can completely remove DNA and RNA from a mixed solution.

A solution of 50 µg/ml DNA in the sodium acetate buffer of Example 13 was prepared by diluting the concentrated DNA solution of Example 13. Four solutions of RNA in the sodium acetate buffer solution were prepared, containing 5, 25, 50 and 100 µg/ml, respectively. Each of these solutions was mixed with an equal volume of the 50 µg/ml DNA solution, and each mixture was divided into 6 samples. Each sample was treated with the amount of Resin B indicated in Table 15, below; the samples were centrifuged at $15,000 \times g$ for two minutes at room temperature, and the absorbance of the supernatants were measured at 260 nm.

As may be seen from Table 15, the removal of light-absorbing material reached a maximum with a concentration of 150 µg/ml of Resin B, and this concentration was independent of the amount of nucleic acids present. In contrast to Example 14 (RNA alone) in which some light-absorbing material remained in the supernatant after treatment with even the optimum amount of resin, no light absorbing material at all remained after treating the mixture containing both DNA and RNA with the optimum amount of resin. The 150 µg/ml treatment level of Resin B was observed to remove 150 µg/ml of mixed nucleic acids.

### Table 15

| Resin concentra-tion (µg/ml) | RNA:DNA ratio | | | |
|---|---|---|---|---|
| | 0.1 | 0.5 | 1.0 | 2.0 |
| | Absorbance at 260 nm | | | |
| 0 | 0.75 | 1.10 | 1.50 | 2.20 |
| 25 | 0.75 | 0.60 | 1.40 | 2.00 |
| 50 | 0.55 | 0.50 | 0.60 | 1.40 |
| 100 | 0.10 | 0.20 | 0.40 | 0.75 |
| 150 | 0.00 | 0.00 | 0.00 | 0.00 |
| 200 | 0.02 | 0.04 | 0.05 | 0.05 |

The term "Waring" as used herein is a trademark which may be registered in some or all of the designated states.

### Claims

1. A method for simultaneously clarifying and stabilizing against the formation of further haze and/or colour a hazy and/or coloured liquid cell-culture medium comprising desired product and one or more of (i) biological cells and/or cell debris, (ii) spent nutrients, and (iii) contaminating water-soluble haze- and/or colour-generating proteinaceous materials, characterized in that the method comprises: –

(1) adding to the cell-culture medium an effective amount of:

(a) particulate polymer having anion, and optionally cation, exchange functionality and a particle diameter of 0.01 to 50 micrometers, and optionally

(b) water-soluble polymer; and

(2) separating solids from the material obtained from step (1) to give a clear, colour and/or haze-free, liquid containing the desired product.

2. A method as claimed in claim 1, wherein the liquid cell-culture medium is aqueous and the particulate polymer is water-insoluble or substantially water-insoluble.

3. A method as claimed in claim 1 or claim 2, wherein the liquid, cell-culture medium comprises microbial cells and/or cell debris.

4. A mehod as claimed in any preceding claim, wherein the desired product comprises antibiotic, protein or carbohydrate.

5. A method as claimed in any preceding claim, wherein the liquid, cell-culture medium comprises cells and/or cell debris of fungus, yeast or bacterium.

6. A method as claimed in any preceding claim, wherein the liquid, cell-culture medium comprises lysate.

7. A method as claimed in any preceding claim, wherein the particulate polymer comprises anion exchange resin.

8. A method as claimed in claim 7, wherein the anion exchange resin comprises approximately spherical beads of crosslinked polymer bearing 0.1 to 1.5 functional groups per monomer unit.

9. A method as claimed in claim 7 or claim 8, wherein the anion exchange resin comprises strongly basic and/or weakly basic anion exchange resin.

10. A method as claimed in any preceding claim, wherein the water-soluble polymer comprises acrylic polymer.

## Revendications

1. Un procédé pour simultanément clarifier et stabiliser contre la formation ultérieure d'un trouble ou d'une coloration un milieu liquide de culture de cellules trouble et/ou coloré comprenant le produit désiré et un ou plusieurs des composants suivants (i) des cellules biologiques et/ou des débris cellulaires, (ii) des substances nutritives usées et (iii) des matières protéiniques contaminantes solubles dans l'eau produisant un trouble et/ou une coloration, ce procédé étant caractérisé en ce qu'il comprend:

(1) l'addition au milieu de culture de cellules d'une quantité efficace de:

(a) un polymère en particules ayant une fonction échangeuse d'anions et facultativement échangeuse de cations et un diamètre des particules de 0,01 à 50 micromètres, et facultativement

(b) un polymère soluble dans l'eau; et

(2) la séparation des solides de la matière obtenue dans le stade (1) pour obtenir un liquide limpide dépourvu de coloration et/ou de trouble contenant le produit désiré.

2. Un procédé selon la revendication 1, dans lequel le milieu liquide de culture de cellules est aqueux et le polymère en particules est insoluble dans l'eau ou essentiellement insoluble dans l'eau.

3. Un procédé selon la revendication 1 ou la revendication 2, dans lequel le milieu liquide de culture de cellules comprend des cellules microbiennes et/ou des débris cellulaires.

4. Un procédé selon l'une quelconque des revendications précédentes, dans lequel le produit désiré comprend un antibiotique, une protéine ou un glucide.

5. Un procédé selon l'une quelconque des revendications précédentes, dans lequel le milieu liquide de culture de cellules comprend des cellules et/ou des débris cellulaires de champignons, de levures ou de bactéries.

6. Un procédé selon l'une quelconque des revendications précédentes, dans lequel le milieu liquide de culture de cellules comprend un lysat.

7. Un procédé selon l'une quelconque des revendications précédentes, dans lequel le polymère en particules comprend une résine échangeuse d'anions.

8. Un procédé selon la revendication 7, dans lequel la résine échangeuse d'anions comprend des perles approximativement sphériques de polymère réticulé portant 0,1 à 1,5 groupe fonctionnel par motif monomère.

9. Un procédé selon la revendication 7 ou 8, dans lequel la résine échangeuse d'anions comprend une résine échangeuse d'anions fortement basique et/ou faiblement basique.

10. Un procédé selon l'une quelconque des revendications précédentes, dans lequel le polymère soluble dans l'eau comprend un polymère acrylique.

## Patentansprüche

1. Verfahren zur gleichzeitigen Klärung und Stabilisierung gegenüber der Bildung einer weiteren Trübung und/oder Färbung eines trüben und/oder gefärbten flüssigen Zellkulturmediums, welches ein gewünschtes Produkt und eines oder mehrere der Bestandteile (i) biologische Zellen und/oder Zelltrümmer, (ii) verbrauchte Nährstoffe und (iii) verunreinigende wasserlösliche, eine Trübung und/oder Verfärbung erzeugende proteinhaltige Materialien aufweist, dadurch gekennzeichnet, daß dieses Verfahren darin besteht,

(1) dem Zellkulturmedium eine wirksame Menge

(a) eines in Form von Einzelteilchen vorliegenden Polymeren mit einer Anionen- und gegebenenfalls Kationenaustauscherfunktionalität und einem Teilchendurchmesser von 0,01 bis 50 μm sowie gegebenenfalls

(b) ein wasserlösliches Polymeres zuzugeben und

(2) die Feststoffe von dem gemäß Stufe (1) erhaltenen Material zur Gewinnung einer klaren, von Verfärbung und/oder Trübung freien Flüssigkeit, welche das gewünschte Produkt enthält, abzutrennen.

2. Verfahren nach Anspruch 1, wobei das Zellkulturmedium wäßrig ist und das in Form von Einzelteilchen vorliegende Polymere in Wasser unlöslich oder im wesentlichen wasserunlöslich ist.

3. Verfahren nach Anspruch 1 oder 2, wobei das flüssige Zellkulturmedium aus Mikrobenzellen oder Zelltrümmern besteht.

4. Verfahren nach einem der vorhergehenden Ansprüche, wobei das gewünschte Produkt aus einem Antibiotikum, Protein oder Kohlehydrat besteht.

5. Verfahren nach einem der vorhergehenden Ansprüche, wobei das flüssige Zellkulturmedium aus Pilz-, Hefe- oder Bakterienzellen und/oder -zelltrümmern besteht.

6. Verfahren nach einem der vorhergehenden Ansprüche, wobei das flüssige Zellkulturmedium aus Lysat besteht.

7. Verfahren nach einem der vorhergehenden Ansprüche, wobei das in Form von Einzelteilchen vorliegende Polymere aus einem Anionenaustauscherharz besteht.

8. Verfahren nach Anspruch 7, wobei das Anionenaustauscherharz aus ungefähr kugelförmigen Kügelchen aus einem vernetzten Polymeren besteht, das 0,1 bis 1,5 funktionelle Gruppen pro Monomereinheit trägt.

9. Verfahren nach Anspruch 7 oder 8, dadurch gekennzeichnet, daß das Anionenaustauscherharz aus stark basischem und/oder schwach basischem Anionenaustauscherharz besteht.

10. Verfahren nach einem der vorhergehenden Ansprüche, wobei das wasserlösliche Polymere aus einem Acrylpolymeren besteht.